# EUROPÄISCHE PATENTSCHRIFT

(11) **EP 0 646 110 B1**
(45) Veröffentlichungstag und Bekanntmachung des Hinweises auf die Patenterteilung: **23.10.1996**
(21) Anmeldenummer: 93912912.8
(22) Anmeldetag: 08.06.1993
(51) Int. Cl.: C07D 209/52, A61K 31/40

(54) **N-SUBSTITUIERTE 3-AZABICYCLO[3.2.0]HEPTAN-DERIVATE ALS NEUROLEPTIKA**
N-SUBSTITUTED 3-AZABICYCLO[3.2.0]HEPTANE DERIVATIVES USEFUL AS NEUROLEPTIC AGENTS
DERIVES N-SUBSTITUES DE 3-AZABICYCLO[3.2.0]HEPTANE UTILES COMME NEUROLEPTIQUES

(30) Priorität: 19.06.1992 DE 4219973
(43) Veröffentlichungstag der Anmeldung: 05.04.1995
(73) Patentinhaber: BASF AKTIENGESELLSCHAFT, 67056 Ludwigshafen (DE)
(72) Erfinder: STEINER, Gerd, D-6719 Kirchheim (DE); UNGER, Liliane, D-6700 Ludwigshafen (DE); BEHL, Berthold, D-6700 Ludwigshafen (DE); TESCHENDORF, Hans-Juergen, D-6724 Dudenhofen (DE)
(86) Internationale Anmeldenummer: EP9301440
(87) Internationale Veröffentlichungsnummer: WO9400431

(56) Entgegenhaltungen:
- WO-A-92/18480
- DE-B- 1 289 845
- US-A- 3 328 390
- US-A- 4 605 655
- JOURNAL OF MEDICINAL CHEMISTRY. Bd. 10, 1967, WASHINGTON US Seiten 621 - 623 CH. H. GROGAN ET AL. 'Omega-azabicyclic butyrophenones'

## Beschreibung

Die Erfindung betrifft neue N-substitierte AzabicycloheptanDerivate, deren Herstellung und Verwendung zur Herstellung von Pharmaka.

Es ist bekannt, daß basisch substituierte Butyrophenon-Derivate bzw. Benzoesäureamid-Derivate Wirkungen als Neuroleptika bzw. Gehirnprotektiva aufweisen (US 4 605 655, EP 410 114, DE 12 89 845, EP 400 661, DE 29 41 880, EP 190 472).

Hierbei scheinen die beobachteten Affinitäten zu σ-Rezeptoren neben den Dopamin- und Serotonin-Affinitäten eine besondere Rolle zu spielen.

Weiter ist bekannt, daß bestimmte Azabicycloalkan-Derivate Wirkungen als Tranquilizer aufweisen (US 3 328 390).

Es wurde nun gefunden, daß N-substituierte 3-Azabicyclo[3.2.0]heptan-Derivate der Formel I 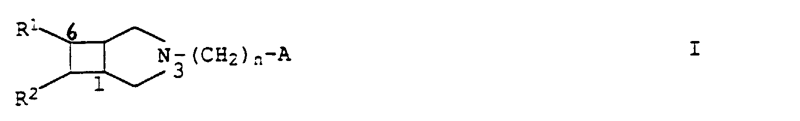 worin
- R¹: eine gegebenenfalls durch Halogenatome, C₁-C₄-Alkyl, Trifluormethyl-, Hydroxy-, C₁-C₄-Alkoxy-, Amino-, Monomethylamino-, Dimethylamino-, Cyano- oder Nitrogruppen mono- oder disubstituierte Phenyl-, Pyridyl-, Thienyl- oder Pyrrolgruppe bedeutet,
- R²: ein Wasserstoffatom oder eine gegebenenfalls durch Halogen, Methoxy, Hydroxy oder Amino substituierte Phenylgruppe ist,
- n: die Zahl 1, 2, 3 oder 4 bedeutet,
- A: ein Wasserstoffatom oder einer der Reste 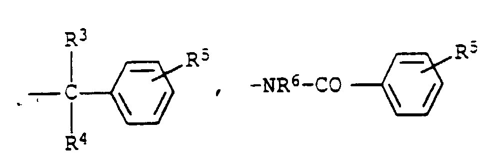 oder -CH=CH₂ ist,
- R³: ein Wasserstoffatom, einen Hydroxyrest oder einen gegebenenfalls durch ein Fluor-, Chlor- oder Bromatom substituierten Phenylrest darstellt,
- R⁴: ein Wasserstoffatom bedeutet oder
- R³ und R⁴: zusammen ein Sauerstoffatom darstellen,
- R⁵: ein Wasserstoff-, Fluor-, Chlor- oder Bromatom oder eine Hydroxy-, Nitro-, C₁-C₄-Alkyl- oder Methoxygruppe bedeutet und
- R⁶: ein Wasserstoffatom oder eine Methylgruppe darstellt,
und deren Salze mit physiologisch verträglichen Säuren wertvolle pharmakologische Eigenschaften besitzen.

In der Formel I haben die Substituenten R¹ bis R⁶ sowie n vorzugsweise folgende Bedeutungen:
- R¹:: Phenyl, gegebenenfalls durch Fluor, Chlor, Methoxy, Trifluormethyl, Nitro, Hydroxy oder Amino substituiert
- R²:: Wasserstoff
- n:: 2 und 3
- R³:: Hydroxy, p-Fluorphenyl
- R⁴:: Wasserstoff oder zusammen mit R³ Sauerstoff
- R⁵:: Wasserstoff, Fluor, Chlor
- R⁶:: Wasserstoff, Methyl.

Die folgenden Verbindungen sind als besonders bevorzugt zu nennen:
1-(4-Fluor-phenyl)-4-[exo-6-phenyl-3-azabicyclo[3.2.0]-heptan3-yl]butan-1-on,
1-(4-Fluor-phenyl)-4-[exo-6-p-fluor-phenyl-3-azabicyclo[3.2.0]heptan-3-yl]butan-1-on,
l-Phenyl-4-[exo-6-phenyl-3-azabicyclo13.2.0]heptan-3-yl]butan-1-on,
1-Phenyl-4-[exo-6-p-fluor-phenyl-3-azabicyclo[3.2.0)heptan3-yl]butan-1-on,
1-(4-Fluor-phenyl)-4-[exo-6-phenyl-3-azabicyclo[3.2.0]heptan3-yl]butan-1-ol,
1-(4-Fluor-phenyl)-4-[exo-6-p-fluor-phenyl-3-azabicyclo[3.2.0]heptan-3-yl]butan-1-ol,
1-Phenyl-4-[exo-6-phenyl-3-azabicyclo[3.2.0]heptan-3-yl]butan-1-ol,
1-Phenyl-4-[exo-6-p-fluor-phenyl-3-azabicyclo[3.2.0]heptan-3-yl]butan-1-ol,
1,1-bis(4-Fluorphenyl)-4-[exo-6-phenyl-3-azabicyclo[3.2.0]heptan-3-yl]butan,
N-(3-[exo-6-Phenyl-3-azabicyclo[3.2.0]heptan-3-yl]-propyl)-4fluor-benzamid,
N-(2-[exo-6-Phenyl-3-azabicyclo[3.2.0]heptan-3-yl]ethyl)-4-fluor-benzamid,
N-(2-[exo-6-Phenyl-3-azabicyclo[3.2.0]heptan-3-yl)-ethyl)N-methyl-4-fluorbenzamid,
N-(2-[exo-6-Phenyl-3-azabicyclo[3.2.0]heptan-3-yl]-ethyl)-N-methyl-benzamid,
N-(3-[exo-6-p-Fluor-phenyl-3-azabicyclo[3.2.0]heptan-3-yl]propyl)-4-fluor-benzamid,
N-(2-[exo-6-p-Fluor-phenyl-3-azabicyclo[3.2.0]heotan-3-yl]ethyl)-benzamid und
N-(2-[ex6-6-p-Fluor-phenyl-3-azabicyclo[3.2.0]heptan-3-yl]ethyl)-N-methyl-benzamid.

Die erfindungsgemäßen Verbindungen der Formel I lassen sich herstellen, indem man eine Verbindung der Formel II

Nu-(CH₂)ₙ-A (II),

in der A und n die angegebenen Bedeutungen haben und Nu eine nukleofuge Abgangsgruppe darstellt, mit einem 3-Azabicyclo[3.2.0]heptan Derivat der Formel III 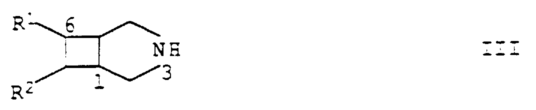 worin
- R¹: eine gegebenenfalls durch Halogenatome, C₁-C₄-Alkyl-, Trifluormethyl, Hydroxy-, C₁-C₄-Alkoxy-, Amino-, Monomethylamino-, Dimethylamino-, Cyano- oder Nitrogruppen mono- oder disubstituierte Phenyl-, Pyridyl-, Thienyl- oder Pyrrolgruppe bedeutet und
- R²: ein Wasserstoffatom oder eine gegebenenfalls durch Halogen, Methoxy, Hydroxy oder Amino substituierte Phenylgruppe ist,
umsetzt und die so erhaltenen Verbindungen gegebenenfalls in ihre Säureadditionssalze mit physiologisch verträglichen Säuren überführt.

Als nukleofuge Abgangsgruppe für Nu kommen vorzugsweise Halogenatome, insbesondere Brom oder Chlor, in Betracht.

Die Umsetzung erfolgt zweckmäßig in Gegenwart einer inerten 3ase, wie Triethylamin oder Kaliumcarbonat, als säurebindendes Mittel in einem inerten Lösungsmittel, wie einem cyclischen gesättigten Ether, insbesondere Tetrahydrofuran oder Dioxan, oder einem Benzolkohlenwasserstoff, wie Toluol oder Xylol.

Die Umsetzung erfolgt in der Regel bei Temperaturen von 20 bis 150°C, und ist im allgemeinen innerhalb von 1 bis 10 Stunden beendet.

Die erfindungsgemäßen Verbindungen der Formel können entweder durch Umkristallisation aus den üblichen organischen Lösungsmitteln, bevorzugt aus einem niederen Alkohol, wie Ethanol, umkristallisiert oder durch Säulenchromatographie gereinigt werden.

Racemate lassen sich in einfacher Weise durch klassische Spaltung mit optisch aktiven Carbonsäuren, z.B. Weinsäure-Derivaten, in einem inerten Lösungsmittel, z.B. niederen Alkoholen, in die Enantiomeren auftrennen.

Die freien 3-Azabicyclo[3.2.0]heptan-Derivate der Formel I können in üblicher Weise in das Säureadditionssalz einer pharmakologisch verträglichen Säure überführt werden, vorzugsweise durch Versetzen einer Lösung mit einem Äquivalent der entsprechenden Säure. Pharmazeutisch verträgliche Säuren sind beispielsweise Salzsäure, Phosphorsäure, Schwefelsäure, Methansulfonsäure, Amidosulfonsäure, Maleinsäure, Fumarsäure, Oxalsäure, Weinsäure oder Zitronensäure.

Die erfindungsgemäßen Verbindungen weisen wertvolle pharmakologische Eigenschaften auf. Sie können als Neuroleptika (insbesondere atypische), Antidepressiva, Sedative, Hypnotika, ZNS-Protektiva oder Muskelrelaxantien Verwendung finden. Mehrere der genannten Wirkungsqualitäten können kombiniert bei einer erfindungsgemäßen Verbindung auftreten. Der pharmakologische Wirkungsnachweis erfolgt sowohl in vivo wie auch in vitro, wobei die Substanzcharakterisierung insbesondere durch die teilweise sehr hohe und selektive Affinität zu Rezeptor-Subtypen, z.B. Dopamin D₁-, D₂-, D₃-, D₄-Rezeptoren; Serotonin 1A-, 1D- und 2-Rezeptoren; Alpha 1- und 2-Rezeptoren; Histamin 1- sowie Muscarin-Rezeptoren, möglich ist.

Für die in vivo Charakterisierung der neuen Substanzen wurden folgende Methoden herangezogen:
a) Beeinflussung der Orientierungsmotilität
   Mäuse zeigen in einer neuen Umgebung ein vermehrtes Explorationsverhalten, das sich in einer gesteigerten motorischen Aktivität außert. Diese motorische Aktivität wird in Lichtschrankenkäfigen für die Zeit von 0 - 30 min nach Einsetzen der Tiere (NMRI-Mäuse, weibl.) in die Käfige gemessen. ED50: Dosis, die die motorische Aktivität im Vergleich zu Placebo-behandelten Kontrollen um 50 % reduziert.
b) Apomorphin-Antagonismus
   Weibliche NMRI-Mäuse erhalten 1,21 mg/kg Apomorphin s.c. Apomorphin führt in dieser Dosis zu einer motorischen Aktivierung, die sich, wenn man die Tiere in Maschendrahtkäfigen hält, in einem permanenten Klettern äußert. Das Klettern wird mit einem Score bewertet (alle 2 min während 30 min):
   0: Tier hat vier Pfoten am Boden
   1: Tier hat zwei Pfoten am Draht
   2: Tier hat vier Pfoten am Draht (klettert).

   Durch Vorbehandlung mit Antipsychotika ist das Kletterverhalten zu hemmen.
   ED50: Dosis, die die Kletteraktivität der Tiere im Vergleich zu Placebo-behandelten Kontrollen um 50 % hemmt.
c) L-5-HTP-Antagonismus
   Weibliche Sprague-Dawley-Ratten erhalten L-5-HTP in einer Dosis von 316 mg/kg i.p. Die Tiere entwickeln darauf ein Erregungssyndrom, von dem Symptome
   - for paw treading und
   - tremor
   mit Hilfe eines Scores (O = nicht vorhanden, 1 = mäßig, 2 = deutlich ausgeprägt) alle 10 min in der Zeit von 20 bis 60 min nach L-5-HTP-Gabe bewertet werden. Im Mittel wird nach L-5-HTP-Gabe ein Score von 17 erreicht. Die Prüfsubstanzen p.o. werden 60 min vor L-5-HTP gegeben. Als ED50 wird die Dosis errechnet, die im Mittel den Kontrollscore um 50 % vermindert.

Die aufgeführten Methoden sind geeignet, Substanzen als Antipsychotika zu charakterisieren. Mit der Hemmung des L-5-HTP-Syndroms kann eine Serotonin-antagonistische Wirkung aufgezeigt werden, eine Wirkungsqualität, wie sie für die sogenannten atypischen Neuroleptika charakteristisch ist.

Die erfindungsgemäßen Substanzen zeigen in diesen Tests eine gute Wirkung.

Die Erfindung betrifft dementsprechend auch ein therapeutisches Mittel, gekennzeichnet durch einen Gehalt an einer Verbindung der Formel I oder deren pharmakologisch verträgliches Saureadditionssalz als Wirkstoff neben üblichen Träger- und Verdünnungsmitteln, sowie die Verwendung der neuen Verbindungen bei der Bekämpfung von Krankheiten.

Die erfindungsgemaßen Verbindungen kannen in üblicher Weise oral oder parenteral, intravenös oder intramuskulär verabfolgt werden.

Die Dosierung hängt vom Alter, Zustand und Gewicht des Patienten sowie von der Applikationsart ab. In der Regel beträgt die tägliche Wirkstoffdosis zwischen etwa 1 und 100 mg/kg Körpergewicht bei oraler Gabe und zwischen 0,1 und 10 mg/kg Körpergewicht bei parenteraler Gabe.

Die neuen Verbindungen können in gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die als Ausgangsstoffe für die Synthese der neuen Verbindungen benötigten Substanzen der Formel II sind bekannt.

Die Substanzen der Formel III lassen sich herstellen, indem man ein Amin der Formel IV 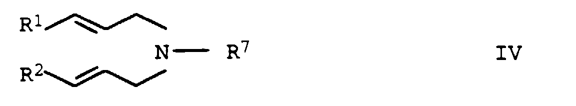 worin R¹ und R² die oben angegebenen Bedeutungen besitzen und R⁷ Wasserstoff, Acetyl, Benzyl oder Trifluoracetyl bedeutet, photochemisch einer 2+2 cycloaddition unterwirft und anschließend gegebenenfalls eine Acyl- oder Benzylgruppe abspaltet.

Die Photoreaktion gelingt gut in einem inerten Lösungsmittel, vorzugsweise Aceton, bei Temperaturen von 20 bis 80°C. Als Lichtquelle eignet sich besonders gut eine Quecksilberhochdrucklampe. Es ist gegebenenfalls vorteilhaft, die Photocycloaddition in einer Quarzapparatur unter einer Stickstoffatmosphäre gegebenenfalls unter Zusatz von etwa 1 Mol Salzsäure pro Mol Amin durchzuführen.

Die Photocycloaddition verläuft in den meisten Fällen hochdiastereoselektiv zu den bicyclischen Verbindungen III mit der exo-Konfiguration bezüglich R¹ und R²:

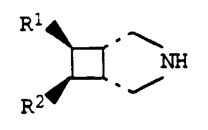

Durch Racematspaltung, z.B. mit optisch aktiven Weinsäure-Derivaten, lassen sich die beiden Enantiomeren rein isolieren.

Die Abspaltung eines Acylrestes (R⁷) erfolgt zweckmäßig durch Verseifung nach bekannten Methoden. Analoges gilt für die Abspaltung des Benzylrestes.

Die Amine der Formel IV sind literaturbekannt oder lassen sich herstellen, indem man entweder einen Aldehyd R¹-CHO mit Vinylmagnesiumchlorid zum Allylalkohol V 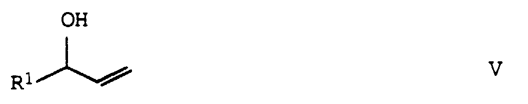 umsetzt, anschließend mit Chlorwasserstoff zum Allylchlorid VI 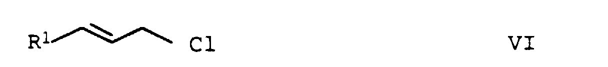 umlagert und zuletzt mit dem entsprechenden Allylamin VII 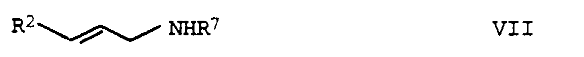 umsetzt, oder man unterzieht einen Zimtaldehyd VIII 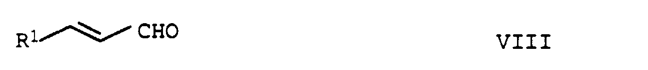 direkt der reduktiven Aminierung mit dem Allylamin VII.

Die folgenden Beispiele dienen zur Erläuterung der Erfindung:

### A) Herstellung der Ausgangsmaterialien

1. exo-6- (p-Fluor) -phenyl-3-azabicyclo[3.2.0]heptan
   19,4 g (102 mM) N-Allyl-N-[3-(4-fluorphenyl)allyl]amin in 130 ml Aceton wurden mit 130 ml 10 %iger Salzsäure sowie mit 600 mg Michlers Keton versetzt und unter Stickstoff 55 h mit einer 150 Watt Quecksilber-Hochdrucklampe in einer Quarzapparatur bei Raumtemperatur bestrahlt. Anschließend engte man den Reaktionsansatz ein und verteilte den Rückstand zwischen Methylenchlorid und Wasser. Man stellte mit wäßriger Ammoniaklösung alkalisch und extrahierte die wäßrige Phase noch zweimal mit Methylenchlorid nach. Die vereinigten organischen Phasen wurden mit Natriumsulfat getrocknet und eingeengt. Ausbeute 19,3 g (99 %), Schmp. 165-166°C (Maleinat).
   Zur Trennung der Antipoden versetzte man 15,0 g (78,5 mM) des Racemats mit einer Lösung von 31,7 g (78,5 mM (-)-Di-O-toluoyl-L-weinsäure in 300 ml siedendem Ethanol. Die beim Abkühlen unter Rühren ausfallenden Kristalle (13,8 g) wurden unter Nachwaschen mit Ethanol abgesaugt und aus 200 ml Ethanol unter Zusatz von 200 ml Wasser umkristallisiert. Freisetzen der Base lieferte den (+)-Antipoden (5,5 g) mit [α]_{D} = + 97,0° (EtOH, c = 0,969).
   Aus der obigen Mutterlauge kristallisierten über Nacht 14,2 g eines Salzes, das aus 400 ml Ethanol (Abfiltrieren des unlöslichen Anteils in der Siedehitze) umkristallisiert wurde (Einengen auf 300 ml). Freisetzen der Base ergab 4,0 g des (-)-Antipoden, [α]_{D} = - 96,0° (EtOH, c = 0,940).
   Die exo-Phenyl-Konfigurationen wurden mittels Röntgenstrukturanalyse nachgewiesen.
2. exo-6-Phenyl-3-azabicyclo[3.2.0]heptan
   50,0 g (28,9 mM) N-Cinnamyl-N-allylamin in 1600 ml Aceton wurden mit 300 ml 10 %iger Salzsäure versetzt und unter Stickstoff 48 h mit einer 150 Watt Quecksilber-Hochdrucklampe in einer Quarzapparatur bei Raumtemperatur bestrahlt. Anschließend engte man den Reaktionsansatz ein und verteilte den Rückstand zwischen Methylenchlorid und Wasser. Man stellte mit wäßriger Ammoniaklösung alkalisch und extrahierte die wäßrige Phase noch zweimal mit Methylenchlorid nach. Die vereinigten organischen Phasen wurden mit Natriumsulfat getrocknet und eingeengt. Ausbeute 49,0 g (98 %) viskoses Öl,
   Schmp. 177 bis 178°C (Maleinat).
3. exo-6,7-Diphenyl-3-benzyl-3-azabicyclo[3.2.0] heptan
   70,0 g (206 mM) Bis-(N-Cinnamyl)-benzylamin in 2500 ml Aceton wurden mit 0,8 g Michlers Keton versetzt und unter Stickstoff 25 h mit einer 150 Watt Quecksilber-Hochdrucklampe in einer Duranglasapparatur bei Raumtemperatur bestrahlt. Anschließend engte man den Reaktionsansatz ein und verteilte den Rückstand zwischen Methylenchlorid und Wasser. Man stellte mit wäßriger Ammoniaklösung alkalisch und extrahierte die wäßrige Phase noch zweimal mit Methylenchlorid nach. Die vereinigten organischen Phasen wurden mit Natriumsulfat getrocknet und eingeengt. Die Reinigung des Rohproduktes (65,0 g) erfolgte durch Säulenchromatographie (Kieselgel, Laufmittel Toluol, Ethanol 98/2). Man erhielt 58,0 g (83 %) Produkt, Schmp.: 230-232°C (Hydrochlorid).
4. exo-6,7-Diphenyl-3-azabicyclo[3.2.0]heptan
   Zu 12,0 g (35,4 mM) exo-6,7-Diphenyl-3-benzyl-3-azabicyclo[3.2.0]heptan in einer Mischung aus 300 ml n-Propanol und 16 ml Wasser wurden 16,0 g (254 mM) Ammoniumformiat sowie 2,0 g Palladium (10 %ig) auf Kohle gegeben und die Reaktionsmischung 4 h am Rückfluß gekocht (Entwicklung von Kohlendioxid). Nach dem Abkühlen saugte man vom Katalysator ab, wusch mit Propanol und Methylenchlorid nach und engte das Filtrat ein. Man verteilte den Rückstand zwischen Methylenchlorid und Wasser, stellte mit wäßriger Ammoniaklösung alkalisch und extrahierte die wäßrige Phase noch zweimal mit Methylenchlorid nach. Die vereinigten organischen Phasen wurden mit Natriumsulfat getrocknet und eingeengt. Man erhielt 8,1 g (92 %) Produkt, Schmp. 140 bis 142°C (Maleinat).
5. exo-6-Phenyl-3-benzyl-3-azabicyclo[3.2.0]heptan
   9,2 g (35,0 mM) N-Cinnamyl-N-allyl-benzylamin in 1100 ml Aceton wurden mit 100 mg Michlers Keton versetzt und unter Stickstoff 5 h mit einer 150 Watt Quecksilber-Hochdrucklampe in einer Duranglasapparatur bei Raumtemperatur bestrahlt. Anschließend engte man den Reaktionsansatz ein. Die Reinigung des Rohproduktes (9,4 g) erfolgte durch Säulenchromatographie (Kieselgel, Laufmittel Methylenchlorid/Methanol 98/2). Man erhielt 3,3 g (36 %) Produkt, Schmp.: 126-128°C (Maleinat).
6. 2,2,2-Trifluoro-l-[exo-6-(3-pyridyl)-3-azabicyclo[3.2.0]hept-3-yl]-ethanon
   14,0 g (51,8 mM) N-Allyl,2,2,2-trifluoro-N-[3-(3-pyridyl)-allyl]-acetamid wurden in 140 ml Aceton gelöst, mit 30 ml 10 %iger wäßriger Salzsäure versetzt und unter Stickstoff 48 h mit einer 150 Watt QuecksilberHochdrucklampe in einer Duranglasapparatur bei Raumtemperatur bestrahlt. Danach wurde die Reaktionslösung eingeengt, in 150 ml Wasser aufgenommen und mit wäßriger Ammoniaklösung auf pH 8-9 eingestellt. Die wäßrige Phase wurde zweimal mit tert.-Butylmethylether extrahiert, die vereinigten organischen Phasen über Natriumsulfat getrocknet und eingeengt. Der verbleibende Rückstand wurde über Säulenchromatographie (Kieselgel, Methylenchlorid + 2 % Methanol) fraktioniert. Man erhielt 6,2 g (42 %) unverändertes N-Allyl-2,2,2-trifluoro-N-[3-(3-pyridyl)allyl]-acetamid und 3,7 g (26 %) 2,2,2-Trifluoro-1-[exo-6-(3-pyridyl)-3-azabicyclo[3.2.0)hept-3-yl]-ethanon als dunkles Öl.
7. exo-6-(3-Pyridyl)-3-azabicyclo[3.2.0]heptan
   Zur Lösung von 3,7 g (13,7 mM) 2,2,2-Trifluoro-1-[exo-6-(3-pyridyl)-3-azabicyclo[3.2.0]hept-3-yl]-ethanon in 50 ml Ethanol wurden 2,5 g Kaliumhydroxid-Plätzchen gegeben. Die Reaktionslösung wurde noch 2 h bei Raumtemperatur nachgerührt und anschließend auf 100 ml Eiswasser gegossen. Die wäßrige Phase wurde dreimal mit tert.-Butyl-methylether extrahiert, die vereinigten organischen Phasen wurden über Natriumsulfat getrocknet und eingeengt. Ausbeute 2,3 (96 %) gelbes Öl, Schmp. 202-205°C (Hydrochlorid).

Analog lassen sich folgende Substanzen herstellen:
8. exo-6-(m-Fluor-phenyl)-3-azabicyclo[3.2.0]heptan
9. exo-6-(o-Fluor-phenyl)-3-azabicyclo[3.2.0]heptan, Schmp. 118-120°C (Maleinat)
10. exo-6- (p-Chlor-phenyl)-3-azabicyclo[3.2.0]heptan, Schmp. 152-154°C (Maleinat)
11. exo-6-(m-Chlor-phenyl)-3-azabicyclo[3.2.0]heptan Schmp. 130-132°C (Maleinat)
12. exo-6- (p-Methoxy-phenyl)-3-azabicyclo[3.2.0]heptan
13. exo-6-(m-Methoxy-phenyl)-3-azabicyclo[3.2.0]heptan
14. exo-6- (p-Nitro-phenyl)-3-azabicyclo[3.2.0]heptan Schmp. 158-160°C (Maleinat)
15. exo-6-(m-Nitro-phenyl)-3-azabicyclo[3.2.0]heptan
16. exo-6-(p-Trifluormethyl-phenyl)-3-azabicyclo[3.2.0]heptan, Schmp. 155-156°C (Maleinat)
17. exo-6-(m-Trifluormethyl-phenyl)-3-azabicyclo[3.2.0]heptan
18. exo-6-(3,4-Dichlor-phenyl)-3-azabicyclo-[3.2.0]heptan
19. exo-6- (3,5-Dichlor-phenyl)-3-azabicyclo-[3.2.0]heptan, Schmp. > 250°C (Hydrochlorid)
20. exo-6-(3,4-Dimethoxy-phenyl))-3-azabicyclo-[3.2.0]heptan
21. exo-6-(m-Hydroxy-phenyl)-3-azabicyclo-[3.2.0]heptan
22. exo-6-(p-Hydroxy-phenyl)-3-azabicyclo-[3.2.0]heptan
23. exo-6-(3,4-Dihydroxy-phenyl)-3-azabicyclo-[3.2.0]heptan
24. exo-6-(p-Methyl-phenyl)-3-azabicyclo-[3.2.0]heptan
25. exo-6-(m-Methyl-phenyl)-3-azabicyclo-[3.2.0]heptan
26. exo-6-(p-t-Butyl-phenyl)-3-azabicyclo-[3.2.0]heptan, Schmp. > 255°C (Hydrochlorid)
27. exo-6-(m-Amino-phenyl)-3-azabicyclo-[3.2.0]heptan
28. exo-6-(p-Amino-phenyl)-3-azabicyclo-[3.2.0]heptan
29. exo-6-(p-Cyano-phenyl)-3-azabicyclo-[3.2.0]heptan, Schmp. 168-170°C (Maleinat)
30. exo-6-Thien-2-yl-3-azabicyclo-[3.2.0]heptan, Schmp. 180-182°C (Hydrochlorid)
31. exo-6-Thien-3-yl-3-azabicyclo-[3.2.0]heptan, Schmp. 143-145°C (Hydrochlorid)
32. exo-6-(5-Chlor-thien-2-yl)-3-azabicyclo-[3.2.0]heptan, Schmp. 156-157°C (Maleinat)
33. exo-6-Pyrrol-2-yl-3-azabicyclo-[3.2.0]heptan
34. exo-6-Pyrid-4-yl-3-azabicyclo-[3.2.0]heptan
35. exo-6-Pyrid-2-yl-3-azabicyclo-[3.2.0]heptan

### B) Herstellung der Endprodukte

### Beispiel 1

### 1-(4-Fluor-phenyl)-4-[exo-6-phenyl-3-azabicyclo[3.2.0]heptan-3-yl]butan-1-on x HCl

8,65 g (50 mM) exo-6-Phenyl-3-azabicyclo[3.2.0]heptan in 130 ml Xylol wurden mit 10,2 ml (60 mM) ω-Chlor-4-fluorbutyrophenon sowie mit 11,5 g (80 mM) fein pulverisiertem Kaliumcarbonat nebst 0,5 g Kaliumiodid versetzt und unter gutem Rühren 7 h unter Rückfluß gekocht.

Nach dem Abkühlen engte man am Rotationsverdampfer ein und verteilte den Rückstand zwischen Methylenchlorid und Wasser.

Die wäßrige Phase wurde zweimal mit Methylenchlorid nachextrahiert und darauf die organische Phase nach einmaligem Nachwaschen mit Wasser und Trocknen mit Natriumsulfat eingeengt. Das Rohprodukt (21 g) reinigte man durch Säulenchromatographie (Kieselgel, Laufmittel Methylenchlorid/ Methanol 94/4). Die freie Base nahm man in 200 ml Ether auf, filtrierte die unlöslichen Flocken ab und versetzte die Etherlösung mit überschüssiger etherischer Salzsäure. Anschließend saugte man die Festkörper in der Kälte ab und wusch das Hydrochlorid mit reichlich Ether nach. Man isolierte 8,3 g (45 %) Produkt, Schmp. 169 bis 171°C.

Analog lassen sich herstellen:
2. 1-Phenyl-4-[exo-6-phenyl-3-azabicyclo[3.2.0]heptan-3-yl]butan-1-on, Schmp.: 134 bis 136°C (Hydrochlorid)
3. 1-(4-Fluor-phenyl)-4-[exo-6,7-diphenyl-3-azabicyclo-[3.2.0]-heptan-3-yl]-butan-1-on, Schmp. 174 bis 176°C (Hydrochlorid)
4. 1-(4-Fluor-phenyl)-4-[exo-6-phenyl-3-azabicyclo[3.2.0]heptan-3-yl]-butan, Schmp. 131 bis 133°C (Hydrochlorid)
5. 1-Phenyl-2-[exo-6-phenyl-3-azabicyclo[3.2.0]heptan-3-yl]ethan-1-on, Öl
6. 1-(4-Fluor-phenyl)-4-[exo-6-p-fluor-phenyl-3-azabicyclo[3.2.0]heptan-3-yl]-butan
7. 1-(4-Fluor-phenyl)-4-[exo-6-m-fluor-phenyl-3-azabicyclo-[3.2.0]heptan-3-yl]-butan-1-on
8. 1-14-Fluor-phenyl)-4-[exo-6-o-fluor-phenyl-3-azabicyclo-[3.2.0]heptan-3-yl]-butan-1-on, Schmp. 180-182°C (Hydrochlorid)
9. 1-(4-Fluor-phenyl)-4-[exo-6-p-chlor-phenyl-3-azabicyclo[3.2.0]heptan-3-yl]-butan-1-on
10. 1-(4-Fluor-phenyl)-4-[exo-6-m-chlor-phenyl-3-azabicyclo[3.2.0]heptan-3-yl]-butan-1-on, Schmp. 137-139°C (Hydrochlorid)
11. 1-(4-Fluor-phenyl)-4-[exo-6-p-methoxy-phenyl-3-azabicyclo[3.2.0]heptan-3-yl]-butan-1-on
12. 1-(4-Fluor-phenyl)-4-[exo-6-m,p-dichlor-phenyl-3-azabicyclo[3.2.0]heptan-3-yl]-butan-1-on,
13. 1-(4-Fluor-phenyl)-4-[exo-6-m,p-dimethoxy-phenyl-3-azabicyclo[3.2.0]heptan-3-yl]-butan-1-on.

### Beispiel 14

### 1-14-Fluor-phenyl)-4-[exo-6-p-fluor-phenyl-3-azabicyclo[3.2.0]heptan-3-yl]-butan-1-on x HCl

4,5 g (23,5 mM) exo-6-p-Fluor-phenyl-3-azabicyclo[3.2.0]heptan in 50 ml Toluol wurden mit 6,0 g (30 mM) -Chlor-4-fluorbutyrophenon sowie mit 4,2 g (30 mM) fein pulverisiertem Kaliumcarbonat nebst 0,5 g Kaliumjodid versetzt und unter gutem Rühren 7 h unter Rückfluß gekocht. Nach dem Abkühlen engte man am Rotationsverdampfer ein und verteilte im Rückstand zwischen Methylenchlorid und Wasser.

Die wäßrige Phase wurde zweimal mit Methylenchlorid nachextrahiert und darauf die organische Phase nach einmaligem Nachwaschen mit Wasser und Trocknen mit Natriumsulfat eingeengt. Das Rohprodukt (9,4 g) reinigte man durch Säulenchromatographie (Kieselgel, Laufmittel Methylenchlorid/Methanol 96/4). Die freie Base nahm man in 150 ml Ether auf, filtrierte die unlöslichen Flocken ab und versetzte die Etherlösung mit überschüssiger etherischer Salzsäure. Nach Zusatz von 10 ml Aceton saugte man die Festkörper in der Kälte ab und wusch das Hydrochlorid mit reichlich Ether nach. Man isolierte 4,9 g (53 %) Produkt, Schmp. 166 bis 168°C.

Analog lassen sich herstellen:
15. 1-Phenyl-4-[exo-6-p-fluor-phenyl-3-azabicyclo[3.2.0]heptan-3-yl]-butan-1-on, Schmp. 141-143°C (Maleinat)
16. 1-14-Fluor-phenyl)-4-[exo-6-p-fluor-phenyl-3-azabicyclo[3.2.0]heptan-3-yl]-butan
17. 1-(4-Fluor-phenyl)-4-[exo-6-p-nitro-phenyl-3-azabicyclo[3.2.0]heptan-3-yl]-butan-1-on, Schmp. 68-70°C (Hydrochlorid)
18. 1-(4-Fluor-phenyl)-4-[exo-6-m-nitro-phenyl-3-azabicyclo[3.2.0]heptan-3-yl]-butan-l-on
19. 1-(4-Fluor-phenyl)-4-[exo-6-p-trifluormethyl-phenyl-3-azabicyclo[3.2.0]heptan-3-yl]-butan-l-on, Schmp. 158-161°C (Hydrochlorid)
20. 1-(4-Fluor-phenyl)-4-[exo-6-p-cyano-phenyl-3-azabicyclo[3.2.0]heptan-3-yl]-butan-1-on
21. 1-(4-Fluor-phenyl)-4-[exo-6-thien-3-yl-3-azabicyclo[3.2.0]heptan-3-yl]-bucan-1-on
22. 1-(4-Fluor-phenyl)-4-[exo-6-pyrid-3-yl-3-azabicyclo[3.2.0]heptan-3-yl]-butan-1-on
23. 1-14-Fluor-phenyl)-3-[exo-6-p-fluor-phenyl-3-azabicyclo[3.2.0]heptan-3-yl]-propan-1-on, Schmp. 151-154°C (Hydrochlorid)

### Beispiel 24

### 1,1- Bis(4-fluor-phenyl)-4-[exo-6-p-fluor-phenyl-3-azabicyclo-[3.2.0]heptan-3-yl]-butan x HCl

5,0 g (25,2 mM) exo-6-p-Fluor-phenyl-3-azabicyclo-[3.2.0]heptan in 80 ml Xylol wurden mit 8,8 g (28, mM) 1,1-(4-Chlorbutyliden)bis-4'-fluorbenzol sowie mit 7,0 g (50,6 mM) fein pulverisiertem Kaliumcarbonat nebst 0,3 g Kaliumjodid versetzt und unter gutem Rühren 15 h unter Rückfluß gekocht. Nach dem Abkühlen engte man am Rotationsverdampfer ein und verteilte den Rückstand zwischen Methylenchlorid und Wasser. Die wäßrige Phase wurde zweimal mit Mtchylenchlorid nachextrahiert und darauf die organische Phase nach einmaligem Nachwaschen mit Wasser und Trocknen mit Natriumsulfat eingeengt. Das Rohprodukt (110 g) reinigte man durch Säulenchromatographie (Kieselgel, Laufmittel Methylenchlorid/Methanol 96/4). Die freie Base nahm man in 350 ml Ether auf, filtrierte die unlöslichen Flocken ab und versetzte die Etherlösung mit überschüssiger etherischer Salzsäure. Einengen lieferte 4,9 g (40 %) Produkt, Schmp. 49 bis 50°C.

Analog läßt sich herstellen:
25. 1,1- Bis(4-fluor-phenyl)-4-[exo-6-phenyl-3-azabicyclo[3.2.0]heptan-3-yl]butan, Schmp. 54 bis 55°C als Hydrochlorid

### Beispiel 26

### 1-(4-Fluor-phenyl)-4-[exo-6-phenyl-3-azabicyclo[3.2.0]heptan-3-yl]-butan-1-ol

4,6 g (13,6 mM) 1-(4-Fluor-phenyl)-4-[exo-6-phenyl-3-azabicyclo[3.2.0]heptan-3-yl]butan-1-on in 60 ml Methanol wurden portionsweise mit 0,6 g (16 mM) Natriumboranat versetzt. Man ließ noch 2 h bei Raumtemperatur nachrühren und engte am Rotationsverdampfer ein. Man verteilte den Kolbenrückstand zwischen Methylenchlorid und Wasser bei pH = 10, extrahierte die wäßrige Phase noch zweimal mit Methylenchlorid, wusch die vereinigten organischen Phasen mit Wasser nach und engte die organische Phase nach dem Trocknen mit Natriumsulfat ein. Das Rohprodukt (4,6 g) reinigte man durch Säulenchromatographie (Kieselgel, Laufmittel Methylenchlorid/Methanol 96/4). Die freie Base nahm man in 150 ml Ether auf, filtrierte die unlöslichen Flocken ab und versetzte die Etherlösung mit überschüssiger etherischer Salzsäure. Anschließend saugte man die Festkörper in der Kälte ab und wusch das Hydrochlorid mit reichlich Ether nach. Man isolierte 3,1 g (61 %) Produkt, Schmp. 147 bis 149°C.

Analog lassen sich herstellen:
27. 1-(4-Fluor-phenyl)-4-[exo-6-p-fluor-phenyl-3-azabicyclo[3.2.0]heptan-3-yl]-butan-l-ol, Schmp. 128 bis 129°C (Hydrochlorid)
28. 1-(4-Fluor-phenyl)-4-[exo-6,7-diphenyl-3-azabicyclo[3.2.0]heptan-3-yl]-butan-l-ol, Schmp. 228 bis 231°C (Hydrochlorid)
29. 1-Phenyl-4-[exo-6-phenyl-3-azabicyclo[3.2.0]heptan-3-yl]butan-1-ol, Schmp. 128 bis 129°C (Hydrochlorid)
30. 1-Phenyl-4-[exo-6-p-fluor-phenyl-3-azabicyclo[3.2.0]heptan-3-yl]-butan-1-ol
31. 1-(4-Fluor-phenyl)-4-[exo-6-o-fluor-phenyl-3-azabicyclo[3.2.0]heptan-3-yl]-butan-1-ol, Schmp. 167-168°C (Hydrochlorid)
32. 1-(4-Fluor-phenyl)-4-[exo-6-m-chlor-phenyl-3-azabicyclo[3.2.0]heptan-3-yl]-butan-1-ol, Schmp. 143-145°C (Hydrochlorid)
33. 1-(4-Fluor-phenyl)-4-[exo-6-p-trifluormethyl-phenyl-3azabicyclo[3.2.0]heptan-3-yl]-butan-1-ol,
   Schmp. 145-148°C (Hydrochlorid)

### Beispiel 34

### 1-(4-Fluor-phenyl)-4-[exo-6-p-amino-phenyl-3-azabicyclo[3.2.0]-heptan-3-yl]-butan-1-on

6,6 g (17,2 mM) 1-(4-Fluor-phenyl)-4-[exo-6-p-nitro-phenyl-3-azabicyclo-[3.2.0]-heptan-3-yl]-butan-1-on wurden in 200 ml Eisessig gelöst, mit 1,7 g Palladium auf Kohle (10 proz.) versetzt und 4 h bei Raumtemperatur und Normaldruck hydriert. Nach Absaugen des Katalysators engte man die Mutterlauge ein, verteilte den Rückstand zwischen Methylenchlorid und Wasser, stellte unter Rühren mit konzentrierter Ammoniak-Lösung alkalisch und extrahierte zweimal mit Methylenchlorid. Nach Trocknen und Einengen der organischen Phase erhielt man 5,0 g Rohprodukt, das durch Säulenchromatographie (Kieselgel, Laufmittel Methylenchlorid/Methanol 95/5) gereinigt wurde. Man isolierte 2,2 g (36 %) 1-(4-Fluor-phenyl)-4-[exo-6-p-amino-phenyl-3-azabicyclo-[3.2.0]-heptan-3-yl]-butan-1-on (Schmp. des Hydrochlorids 136-139°C) und 1,4 g (23 %) 1-(4-Fluor-phenyl)-4-[exo-6-p-amino-phenyl-3-azabicyclo[3.2.0]-heptan-3-yl]-butan-1-ol (Schmp. des Hydrochlorids 122-125°C).

### Beispiel 35

### N-(3-[exo-6-Phenyl-3-azabicyclo[3.2.0]heptan-3-yl]-propyl)-4-fluor-benzamid

3,5 g (20 mM) exo-6-Phenyl-3-azabicyclo[3.2.0]heptan in 40 ml Toluol wurden mit N-(3-Chlorpropyl)-4-fluor-benzamid sowie mit 4,8 g (35 mM) fein pulverisiertem Kaliumcarbonat nebst 0,5 g Kaliumjodid versetzt und unter gutem Rühren 9 h unter Rückfluß gekocht. Nach dem Abkühlen engte man am Rotationsverdampfer ein und verteilte den Rückstand zwischen Methylenchlorid und Wasser. Die wäßrige Phase wurde zweimal mit Methylenchlorid nachextrahiert und darauf die organische Phase nach dem Trocknen mit Natriumsulfat eingeengt. Das Rohprodukt (8,4 g) reinigte man durch Säulenchromatographie (Kieselgel, Laufmittel Methylenchlorid/Methanol 96/4). Man löste die gereinigte freie Base in einer Mischung aus 100 ml Ether und 10 ml Aceton und tropfte unter Eiskühlung und Rühren eine Lösung von 1,6 g Maleinsäure in Aceton langsam zu. Das ausgefallene Salz wurde unter Stickstoff abgesaugt, mit Ether nachgewaschen und unter Stickstoff getrocknet. Man isolierte 4,9 g (70 %) hygroskopisches Produkt als Maleinatsalz, Schmp. 122 bis 124°C.

In analoger Weise lassen sich herstellen:
36. N-(3-[exo-6-Phenyl-3-azabicyclo[3.2.0]heptan-3-yl]propyl)-benzamid, Schmp. 70 bis 72°C (Hydrochlorid)
37. N-(2-[exo-6-Phenyl-3-azabicyclo[3.2.0]heptan-3-yl]ethyl)-4-fluor-benzamid
38. N-(2-[exo-6-Phenyl-3-azabicyclo[3.2.0]heptan-3-yl]ethyl)-benzamid, Schmp. 89 bis 90°C
39. N-(2-[exo-6-Phenyl-3-azabicyclo[3.2.0]heptan-3-yl]ethyl)-N-methyl-4-fluorbenzamid, Schmp. 126 bis 128°C (Hydrochlorid)
40. N-(2-[exo-6-Phenyl-3-azabicyclo[3.2.0]heptan-3-yl]ethyl)-N-methyl-benzamid, Schmp. 121 bis 122°C (Hydrochlorid)
41. N-(2-[exo-6-Phenyl-3-azabicyclo[3.2.0]heptan-3-yl]ethyl)-N-methyl-4-isopropylbenzamid, Schmp. 184 bis 185°C (Hydrochlorid)
42. N-(3-[exo-6-Phenyl-3-azabicyclo[3.2.0]heptan-3-yl]propyl)-4-chlor-benzamid
43. N-(2-[exo-6-p-Trifluormethyl-phenyl-3-azabicyclo[3.2.0]heptan-3-yl]-ethyl)-4-chlor-benzamid, Schmp. 112-114°C
44. N-(3-[exo-6-Phenyl-3-azabicyclo[3.2.0]heptan-3-yl]propyl)-3-methoxy-benzamid
45. N-(3-[exo-6-Phenyl-3-azabicyclo[3.2.0]heptan-3-yl]propyl)-3-nitro-benzamid
46. N-(3-[exo-6-p-Fluor-phenyl-3-azabicyclo[3.2.0] heptan-3-yl]-propyl)-4-fluor-benzamid, Schmp. 160 bis 162°C (Hydrochlorid)
47. N-(3-[exo-6-p-Fluor-phenyl-3-azabicyclo[3.2.0]heptan-3-yl]-propyl)-benzamid, Schmp. 177 bis 178°C (Hydrochlorid)
48. N-(2- [exo-6-p-Fluor-phenyl-3-azabicyclo[3.2.0]heptan-3-yl]-ethyl)-4-fluor-benzamid, Schmp. 111 bis 113°C
49. N-(2-[exo-6-p-Fluor-phenyl-3-azabicyclo[3.2.0]heptan-3-yl]-ethyl)-benzamid, Schmp. 94 bis 95°C
50. N-(2-[exo-6-p-Fluor-phenyl-3-azabicyclo[3.2.0]heptan-3-yl]-ethyl)-N-methyl-4-fluor-benzamid, Schmp. 170 bis 171°C (Hydrochlorid)
51. N-(2-[exo-6-p-Fluor-phenyl-3-azabicyclo[3.2.0]heptan-3-yl]-ethyl)-N-methyl-benzamid
52. N- (2-[exo-6-p-Fluor-phenyl-3-azabicyclo[3.2.0]heptan-3-yl]-ethyl)-N-methyl-4-isopropylbenzamid, Schmp. 189 bis 190°C (Hydrochlorid)
53. N-(3-[exo-6-p-Fluor-phenyl-3-azabicyclo[3.2.0]heptan-3-yl]-propyl)-4-chlor-benzamid
54. N-(3-[exo-6-p-Fluor-phenyl-3-azabicyclo[3.2.0] heptan-3-yl]-propyl)-3-methoxy-benzamid
55. N-(3-[exo-6-p-Fluor-phenyl-3-azabicyclo[3.2.0] heptan-3-yl]-propyl)-3-nitro-benzamid.
56. N-(2-[exo-6-m-Chlor-phenyl-3-azabicyclo[3.2.0]heptan-3-yl]-ethyl)-4-chlor-benzamid, Schmp. 96-98°C
57. N-(2-[exo-6-o-Fluor-phenyl-3-azabicyclo[3.2.0]heptan-3-yl]-ethyl)-4-chlor-benzamid, Schmp. 91-93°C
58. N-(2-[exo-6-p-Fluor-phenyl-3-azabicyclo[3.2.0]heptan-3-yl]-ethyl)-2-hydroxy-benzamid, Schmp. 93-95°C (siehe auch Beispiel 59)

### Beispiel 59

### N-(2-[exo-6-m-Hydroxy-phenyl-3-azabicyclo[3.2.0]heptan-3-yl]ethyl)-4-chlor-benzamid

Zu 4,2 g (11 mM) N-(2-[exo-6-m-Methoxy-phenyl-3-azabicyclo[3.2.0]heptan-3-yl]-ethyl)-4-chlor-benzamid in 70 ml Methylenchlorid wurden 13 ml (13 mM) Bortribromid (1 M Lösung in Methylenchlorid) bei Raumtemperatur getropft und das Gemisch über Nacht gerührt. Nach Kühlen wurden 100 ml 2 n Ammoniumhydroxidlösung hinzugegeben, die organische Phase abgetrennt und die wäßrige Phase mit Methylenchlorid nachextrahiert. Nach Trocknen und Einengen erhielt man 4,5 g Rohprodukt, das durch Säulenchromatographie (Kieselgel, Laufmittel Methylenchlorid/Methanol 95/5) gereinigt wurde. Man isolierte 2,8 g (69 %) Produkt, Schmp. 65-68°C.

### Beispiel 60

### exo-3-n-Butyl-6-phenyl-3-azabicyclo[3.2.0]heptan Maleinat

3,5 g (20 mM) exo-6-phenyl-3-azabicyclo[3.2.0]heptan in 50 ml Tetrahydrofuran wurden mit 4,2 ml (30 mM) Triethylamin sowie mit 5,4 g (40 mM) n-Butylbromid versetzt und unter gutem Rühren 9 h unter Rückfluß gekocht.

Nach dem Abkühlen engte man am Rotationsverdampfer ein und verteilte den Rückstand zwischen Methylenchlorid und Wasser.

Die wäßrige Phase wurde zweimal mit Methylenchlorid nachextrahiert und darauf die organische Phase nach dem Trocknen mit Natriumsulfat eingeengt. Das Rohprodukt (4,2 g) reinigte man durch Säulenchromatographie (Kieselgel, Laufmittel Methylenchlorid/Methanol 96/4). Die gereinigte freie Base (3,1 g) löste man in 200 ml Ether und tropfte unter Eiskühlung und Rühren die stöchiometrische Menge Maleinsäure in Aceton langsam zu. Das ausgefallene Salz wurde unter Stickstoff abgesaugt, mit Ether nachgewaschen und unter Stickstoff getrocknet. Man isolierte 4,4 g (64 %) Maleinatsalz, Schmp. 125 bis 126°C.

In analoger Weise lassen sich herstellen:
61. exo-3-Methyl-6-phenyl-3-azabicyclo[3.2.0]heptan, Schmp. 129-131°C (Maleinat)
62. exo-3-Methyl-6-p-fluor-phenyl-3-azabicyclo[3.2.0]heptan
63. exo-3-n-Propyl-6-p-fluor-phenyl-3-azabicyclo[3.2.0]heptan
64. exo-3-Methyl-6,7-diphenyl-3-azabicyclo[3.2.0]heptan, Schmp. 197 bis 198°C (Hydrochlorid).
65. exo-3-n-Propyl-6-m-hydroxy-phenyl-3-azabicyclo[3.2.0]heptan (siehe auch Beispiel 59), Schmp. 148-150°C (Hydrochlorid)
66. exo-3-Allyl-6-m-methoxy-phenyl-3-azabicyclo[3.2.0]heptan, Schmp. 118-120°C (Hydrochlorid)
67. exo-3-Phenethyl-6-p-fluor-phenyl-3-azabicyclo[3.2.0]heptan, Schmp. 128-129°C (Maleinat)

## Patentansprüche

1. N-substituierte 3-Azabicyclo[3.2.0]-heptan-Derivate der Formel I 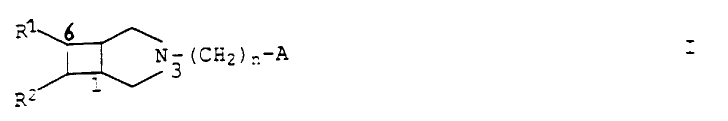 worin
R¹ eine gegebenenfalls durch Halogenatome, C₁-C₄-Alkyl, Trifluormethyl-, Hydroxy-, C₁-C₄-Alkoxy-, Amino-, Monomethylamino-, Dimethylamino-, Cyano- oder Nitrogruppen mono- oder disubstituierte Phenyl-, Pyridyl-, Thienyl- oder Pyrrolgruppe bedeutet,
R² ein Wasserstoffatom oder eine gegebenenfalls durch Halogen, Methoxy, Hydroxy oder Amino substituierte Phenylgruppe ist,
n die Zahl 1, 2, 3 oder 4 bedeutet,
A ein Wasserstoffatom oder einer der Reste 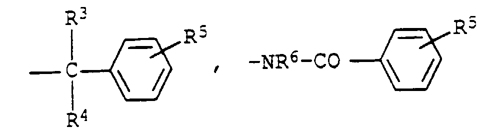 oder -CH=CH₂ ist,
R³ ein Wasserstoffatom, einen Hydroxyrest oder einen gegebenenfalls durch ein Fluor-, Chlor- oder Bromatom substituierten Phenylrest darstellt,
R⁴ ein Wasserstoffatom bedeutet oder
R³ und R⁴ zusammen ein Sauerstoffatom darstellen,
R⁵ ein Wasserstoff-, Fluor-, Chlor- oder Bromatom oder eine hydroxy-, Nitro-, C₁-C₄-Alkyl- oder Methoxygruppe bedeutet und
R⁶ ein Wasserstoffatom oder eine Methygruppe darstellt,
und deren Salze mit physiologisch verträglichen Säuren.

2. N-substituerte 3-Azabicyclo[3.2.0]heptan-Derivate der Formel I gemäß Anspruch 1 zur Verwendung bei der Bekämpfung von Krankheiten.

## Claims

1. An N-substituted 3-azabicyclo[3.2.0]heptane derivative of the formula I 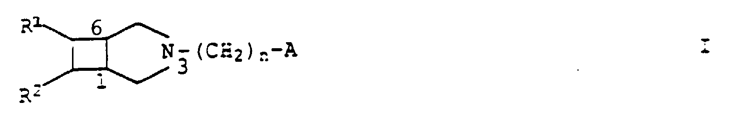 where
R¹ is a phenyl, pyridyl ,thienyl or pyrrrolyl group which is unsubstituted or mono- or disubstituted by halogen atoms, C₁-C₄-alkyl, trifluoromethyl, hydroxyl, C₁-C₄-alkoxy, amino, monoethylamino, dimethylamino, cyano or nitro groups,
R² is a hydrogen atom or a phenyl group which is unsubstituted or substituted by halogen, methoxy, hydroxyl or amino
n is the number 1, 2, 3 or 4,
A is a hydrogen atom or one of the radicals 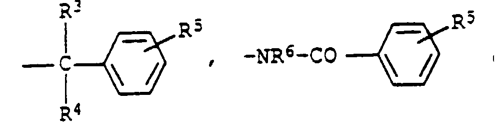 or -CH=CH₂
R³ is a hydrogen atom, a hydroxyl radical or a phenyl radical which is unsubstituted or substituted by a fluorine, chlorine or bromine atom,
R⁴ is a hydrogen atom, or
R³ and R⁴ together represent an oxygen atom,
R⁵ is a hydrogen, fluorine, chlorine or bromine atom or a hydroxyl, nitro, C₁-C₄-alkyl or methoxy group, and
R⁶ is a hydrogen atom or a methyl group,
and salts thereof with physiologically tolerated acids.

2. An N-substituted 3-azabicyclo [3.2.0] heptane derivative of the formula I as claimed in claim 1 for use for controlling diseases.

## Revendications

1. Dérivés substitués à l'azote du 3-azabicyclo[3.2.0]heptane, de formule I 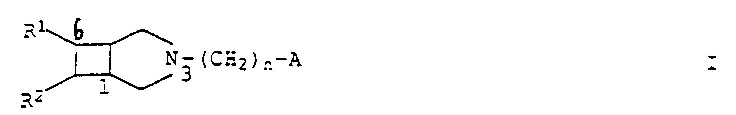 dans laquelle
R¹ représente un groupe phényle, pyridyle, thiényle ou pyrrole éventuellement mono- ou di-substitué par des atomes d'halogènes, des groupes alkyle en C1-C4, trifluorométhyle, hydroxy, alcoxy en C1-C4, amino, monométhylamino, diméthylamino, cyano ou nitro,
R² représente un atome d'hydrogène ou un groupe phényle éventuellement substitué par des halogènes, des groupes méthoxy, hydroxy ou amino,
n est égal à 1, 2, 3 ou 4,
A représente un atome d'hydrogène ou l'un des groupes 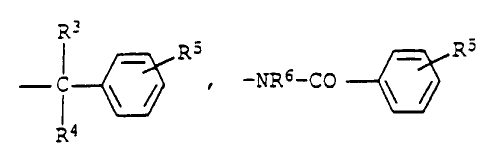 ou -CH=CH₂
R³ représente un atome d'hydrogène, un groupe hydroxy ou un groupe phényle éventuellement substitué par un atome de fluor, de chlore ou de brome,
R⁴ représente un atome d'hydrogène, ou bien
R³ et R⁴ représentent ensemble un atome d'oxygène,
R⁵ représente un atome d'hydrogène, de fluor, de chlore ou de brome ou un groupe hydroxy, nitro, alkyle en C1-C4 ou méthoxy et
R⁶ représente un atome d'hydrogène ou un groupe méthyle,
et leurs sels d'acides acceptables pour l'usage pharmaceutique.

2. Dérivés substitués à l'azote du 3-azabicyclo[3.2.0]heptane de formule I de la revendication 1, pour l'utilisation dans le traitement de maladies.
